(19) Europäisches Patentamt
European Patent Office
Office européen des brevets

(11) Veröffentlichungsnummer : **0 047 941**
**B1**

(12) # EUROPÄISCHE PATENTSCHRIFT

(45) Veröffentlichungstag der Patentschrift : **07.12.83**

(51) Int. Cl.³ : **C 07 D233/60**, C 07 D249/08

(21) Anmeldenummer : **81106979.8**

(22) Anmeldetag : **05.09.81**

(54) **Verfahren zur Herstellung und Reinigung von Phenoxy-azolyl-butanon-Derivaten.**

(30) Priorität : **17.09.80 DE 3035022**

(43) Veröffentlichungstag der Anmeldung : **24.03.82 Patentblatt 82/12**

(45) Bekanntmachung des Hinweises auf die Patenterteilung : **07.12.83 Patentblatt 83/49**

(84) Benannte Vertragsstaaten : **BE CH DE FR GB IT LI NL**

(56) Entgegenhaltungen :
**EP-A- 0 011 191**
**AT-B- 358 031**
**DE-A- 2 743 767**
**DE-B- 2 201 063**
**DE-B- 2 406 665**
**Die Akte enthält technische Angaben, die nach dem Eingang der Anmeldung eingereicht wurden und die nicht in dieser Patentschrift enthalten sind.**

(73) Patentinhaber : **BAYER AG**
**Zentralbereich Patente, Marken und Lizenzen**
**D-5090 Leverkusen 1, Bayerwerk (DE)**

(72) Erfinder : **Elbe, Hans-Ludwig, Dr.**
**Dasnoeckel 59**
**D-5600 Wuppertal 11 (DE)**
Erfinder : **Arold, Hermann, Dr.**
**Falkenberg 151**
**D-5600 Wuppertal 1 (DE)**
Erfinder : **Kranz, Eckart, Dr.**
**Am Acker 9**
**D-5600 Wuppertal 1 (DE)**
Erfinder : **Stölzer, Claus, Dr.**
**Sillerstrasse 44**
**D-5600 Wuppertal 11 (DE)**

**EP 0 047 941 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

Verfahren zur Herstellung und Reinigung von Phenoxy-azolyl-butanon-Derivaten

Die vorliegende Erfindung betrifft ein neues, technisch einsetzbares Verfahren zur Herstellung und Reinigung von bekannten Phenoxy-azolyl-butanon-Derivaten.

Es ist bereits bekannt geworden, daß man Phenoxy-azolyl-butanon-Derivate erhalten kann, wenn man Dihalogenpinakoline mit 1,2,4-Triazol und Phenolen in Gegenwart eines Säurebindemittels, wie z. B. Kaliumcarbonat, und in Gegenwart eines polaren Lösungsmittels, wie z. B. Aceton, bei Temperaturen zwischen 0 und 150 °C umsetzt (vergleiche DE-AS 24 06 665).

Dieses Verfahren hat jedoch den Nachteil, daß insbesondere bei der Herstellung im technischen Maßstab Konkurrenzreaktionen zur verstärkten Bildung von Nebenprodukten führen, die den Gehalt an gewünschtem Endprodukt beträchtlich verringern. Außerdem ist aufgrund einer umständlichen und zeitraubenden Aufarbeitung die Raum/Zeit-Ausbeute unbefriedigend.

Weiterhin ist ein Verfahren bekannt, welches nicht von Dihalogenpinakolin, sondern vom Monochlorpinakolin ausgeht, wobei durch Umsetzung mit einem Phenol zuerst ein Phenoxyetherketon erhalten wird, welches dann durch eine Halogenierung in ein Phenoxyhalogenetherketon überführt wird ; die letztgenannte Verbindung läßt sich sodann mit 1,2,4-Triazol zu dem gewünschten Endprodukt umsetzen (vgl. hierzu die AT-PS 358 031). Der Nachteil dieses Verfahrens ist, daß die Gesamtsynthese sich als relativ zeit- und arbeitsintensiv erweist.

Es wurde nun gefunden, daß man die bekannten Phenoxyazolyl-butanon-Derivate der allgemeinen Formel

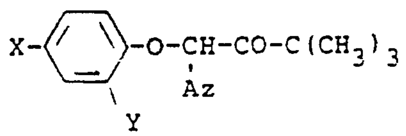

(I)

in welcher
X für Halogen steht,
Y für Wasserstoff oder Halogen steht und
Az für einen Imidazolyl- oder 1,2,4-Triazolyl-Rest steht,
durch Umsetzung von Dichlorpinakolin der Formel

$$Cl_2CH—CO—C(CH_3)_3 \qquad (II)$$

mit einem Azol der allgemeinen Formel

$$Az—H \qquad (III)$$

in welcher
Az die weiter oben angegebene Bedeutung besitzt,
und einem Phenol der Formel

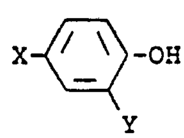

in welcher
X und Y die weiter oben angegebene Bedeutung besitzen,
in Gegenwart eines Säurebindemittels auch in technischem Maßstab mit guter Ausbeute und Reinheit dann erhält, wenn man die Umsetzung in Gegenwart eines mit Wasser nicht mischbaren organischen Lösungsmittels bei Temperaturen zwischen 40 und 150 °C durchführt, das rohe Endprodukt der Formel I ohne Lösungsmittelwechsel in Lösung beläßt und zur Reinigung in der gleichen Lösung bei Temperaturen zwischen 0 und + 80 °C mit Mineralsäuren versetzt und aus dem abgetrennten Salz das reine Endprodukt der Formel I in üblicher Weise isoliert.

Es ist als ausgesprochen überraschend zu bezeichnen, daß die Phenoxy-azolyl-butanon-Derivate der Formel I, die ja bekanntlich zur Gruppe der O,N-Acetale gehören, in Gegenwart von Mineralsäure unter den Bedingungen der Fällung (bei 0 bis 80 °C) stabil sind, da allgemein bekannt ist, daß O,N-Acetale durch Säurekatalyse leicht gespalten werden können. Dies ist umso überraschender, da das Salz der Verbindung der Formel I nicht momentan ausfällt, sondern dem Angriff überschüssiger Mineralsäure einige Zeit ausgesetzt ist.

Ebenfalls überraschend ist, daß die für die erfindungsgemäße Verfahrensweise als organische Lösungsmittel vorzugsweise zu verwendenden mit Wasser nicht mischbaren Ketone für die Salzfällung eingesetzt werden können, wie z. B. das Methyl-isobutyl-keton. Es ist nämlich allgemein bekannt, daß z. B. Aceton als ein mit dem Methylisobutyl-keton strukturell vergleichbares Lösungsmittel unter dem

katalytischen Einfluß von Mineralsäuren, wie insbesondere von der erfindungsgemäß vorzugsweise zu verwendenden Schwefelsäure, sehr leicht Kondensationsreaktionen eingeht. Die Stabilität von Methyl-isobutyl-keton in Gegenwart von konzentrierter Schwefelsäure im anzuwendenden Temperaturbereich von 0 bis 80 °C war daher kaum zu erwarten.

Die erfindungsgemäße Verfahrensdurchführung hat den Vorteil, daß das gewünschte Produkt auch bei technischer Herstellung mit eines Gehalt von > 97 % erhalten wird bei gleichzeitiger guter Raum/Zeit-Ausbeute.

In der allgemeinen Formel I steht X vorzugsweise für Chlor, Y vorzugsweise für Wasserstoff oder Chlor, und Az vorzugsweise für den 1,2,4-Triazol-1-yl-Rest.

Eine bevorzugte Verfahrensdurchführung kann wie folgt vorgenommen werden :

1 bis 1,5 KMol Dichlorpinakolin und 1 bis 4 KMol Säurebinder werden in einem mit Wasser nicht mischbaren Keton (wie z. B. Methyl-isobutyl-keton) oder einem aromatischen Kohlenwasserstoff (wie z. B. Toluol) mit einer Mischung aus 1 bis 1,5 KMol Azol (wie z. B. 1,2,4-Triazol oder Imidazol) und 1 KMol eines Phenols (wie z. B. 4-Chlorphenol) bei 40 bis 120 °C zur Reaktion gebracht. Diese Art der Zugabe ist bevorzugt ; es ist aber auch möglich, das Phenol, das Azol und den Säurebinder vorzulegen und Dichlorpinakolin zuzugeben. Die Reaktionszeit beträgt 8 bis 15 Stunden. Zur Aufarbeitung versetzt man das Reaktionsgemisch mit Wasser und trennt anschließend die Phasen. Die organische Phase, die das gewünschte Reaktionsprodukt enthält, wird zur weiteren Entfernung von unerwünschten Nebenprodukten mit verdünnter Natronlauge gewaschen. Nach erneuter Phasentrennung wird die organische Phase mit Mineralsäure, wie z. B. Schwefelsäure, bei 0 bis 80 °C versetzt.

Aus dem abgetrennten Salz wird die freie Base der Formel (I) in üblicher Weise mit einem Gehalt von > 97 % (gaschromatographisch bestimmt) erhalten.

Als mit Wasser nicht mischbare organische Lösungsmittel kommen insbesondere in Frage : Methylisobutylketon oder andere mit Wasser nicht mischbare Ketone.

Es ist weiterhin auch möglich, andere mit Wasser nicht mischbare Lösungsmittel zu verwenden, wie z. B. aromatische Kohlenwasserstoffe. Als Beispiele sind zu nennen : Toluol, Xylol und Chlorbenzol.

Als Säurebinder kommen für die Umsetzung alle üblichen organischen und insbesondere anorganischen Säurebindemittel in Frage. Beispielsweise seien genannt : tertiäre Amine, wie Triethylamin oder Dimethylcyclohexylamin ; Alkalihydroxide, wie Natrium- und Kaliumhydroxid ; Alkalicarbonate, wie Natrium- und Kaliumcarbonat ; sowie Erdalkalihydroxide und -carbonat, wie Calciumhydroxid und Calciumcarbonat.

Als für das erfindungsgemäße Verfahren für die Salzabtrennung benötigte Mineralsäure wird vorzugsweise Schwefelsäure, wie z. B. konzentrierte Schwefelsäure, verwendet.

Die Umsetzung wird bei Temperaturen zwischen 40 und 150 °C, vorzugsweise zwischen 40 und 120 °C durchgeführt.

Der erfindungsgemäße Reinigungsschritt wird bei Temperaturen zwischen 0 und 80 °C, vorzugsweise zwischen 20 und 70 °C, durchgeführt.

Bei der Durchführung der Umsetzung setzt man auf 1 KMol Phenol vorzugsweise 1 bis 1,5 KMol Dichlorpinakolin, 1 bis 1,5 KMol Azol und 1 bis 4 KMol Säurebinder ein.

Die Verbindungen der Formel (I) weisen eine sehr gute fungizide Wirksamkeit auf (vergleiche DE-AS 22 01 063). Sie können beispielsweise mit besonders gutem Erfolg als Mittel gegen echten Mehltau (als Blattfungizid) und gegen Erkrankungen des Getreides, wie Getreiderost (als Saatgutbeizmittel) verwendet werden.

Das erfindungsgemäße Verfahren — im Vergleich zum Stand der Technik — sei anhand des folgenden Herstellungsbeispiels erläutert :

Herstellungsbeispiele im technischen Maßstab

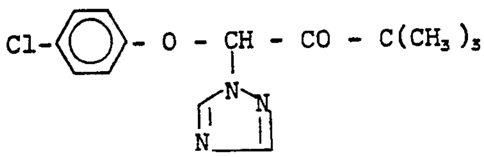

a) erfindungsgemäß

In einem 3 000 l-Rührkessel werden 197 kg (1 165 Mol) Dichlorpinakolin und 420 kg (3 040 Mol) Kaliumcarbonat in 640 kg Methyl-isobutyl-keton auf 90 °C erwärmt. Dazu wird ein Gemisch aus 129 kg (1 000 Mol) 4-Chlorphenol und 76,4 kg (1 100 Mol) 1,2,4-Triazol gegeben. Man läßt 10 Stunden bei 90 bis 95 °C nachrühren, kühlt auf 50 °C ab und versetzt mit 1 400 kg Wasser. Man rührt 30 Minuten nach und trennt anschließend die wässrige Phase ab. Die organische Phase wird mit 350 kg verdünnter Natronlauge und danach mit 50 kg Wasser gewaschen. Bei 40 °C läßt man 76 kg 96 %-ige Schwefelsäure einlaufen und kühlt danach auf 10 °C ab. Der ausgefallene Niederschlag wird mit 300 kg Methyl-isobutyl-keton gewaschen und in einem Gemisch aus 400 kg Methyl-isobutyl-keton, 400 kg Wasser und 100 kg

45 %-iger Natronlauge hydrolysiert. Die wässrige Phase wird abgetrennt, die organische Phase eingeengt. Man erhält 183 kg (61 % der Theorie, bezogen auf eingesetztes 4-Chlorphenol) 1-(4-Chlorphenoxy)-3,3-dimethyl-1-(1,2,4-triazol-1-yl)-butan-2-on vom Schmelzpunkt 75-76 °C mit einem Gehalt von 97,5 % (gaschromatographisch bestimmt).

b) bekannt (Ansatz in technischem Maßstab in Anlehnung an die Vorschrift in der DE-AS 24 06 665)

In einem 3 000 l-Rührkessel werden 550 kg Aceton vorgelegt. Unter Rühren werden 104 kg (1 500 Mol) 1,2,4-Triazol, 634 kg (4 600 Mol) Kaliumcarbonat sowie 193 kg (1 500 Mol) 4-Chlorphenol hinzugegeben. Man erhitzt auf Rückfluß und versetzt mit 330 kg (1 950 Mol) Dichlorpinakolin, gelöst in 240 kg Aceton. Nach beendeter Zugabe wird 16 Stunden unter Rückfluß gerührt. Man läßt auf Raumtemperatur abkühlen und filtriert. Das Filtrat wird durch Abdestillieren des Lösungsmittels eingeengt. Der Rückstand wird in 1 300 kg Toluol aufgenommen, zunächst mit einem Gemisch aus 250 kg Wasser und 134 kg konzentrierter Salzsäure und danach nochmals mit 400 kg Wasser gewaschen. Nach dem Einengen erhält man 408 kg (61 % der Theorie, bezogen auf eingesetztes 4-Chlorphenol) 1-(4-Chlorphenoxy)-3,3-dimethyl-1-(1,2,4-triazol-1-yl)-butan-2-on als zähflüssiges Oel mit einem Gehalt von 66 % (gaschromatographisch bestimmt).

## Ansprüche

1. Verfahren zur Herstellung von Phenoxy-azolyl-butanon-Derivaten der allgemeinen Formel

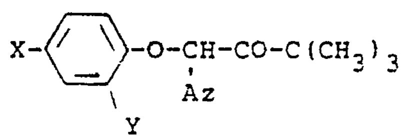

(I)

in welcher
X für Halogen steht,
Y für Wasserstoff oder Halogen steht und
Az für einen Imidazolyl- oder 1,2,4-Triazolyl-Rest steht,
durch Umsetzung von Dichlorpinakolin der Formel

$$Cl_2CH—CO—C(CH_3)_3 \quad (II)$$

mit einem Azol der allgemeinen Formel

$$Az—H \quad (III)$$

in welcher
Az die weiter oben angegebene Bedeutung besitzt
und einem Phenol der Formel

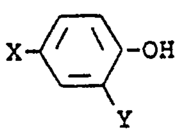

in welcher
X und Y die weiter oben angegebene Bedeutung besitzen,
in Gegenwart eines Säurebindemittels, dadurch gekennzeichnet, daß man die Umsetzung in Gegenwart eines mit Wasser nicht mischbaren organischen Lösungsmittels bei Temperaturen zwischen 40 und 150 °C durchführt, das rohe Endprodukt der Formel I ohne Lösungsmittelwechsel in Lösung beläßt und zur Reinigung in der gleichen Lösung bei Temperaturen zwischen 0 und + 80 °C mit Mineralsäuren versetzt und aus dem abgetrennten Salz das reine Endprodukt der Formel I in üblicher Weise isoliert.

2. Verfahren gemäß Anspruch 1, dadurch gekennzeichnet, daß man die Umsetzung in Gegenwart eines mit Wasser nicht mischbaren Ketons durchführt.

3. Verfahren gemäß Ansprüchen 1 und 2, dadurch gekennzeichnet, daß man die Umsetzung in Gegenwart von Methyl-isobutyl-keton durchführt.

4. Verfahren gemäß Anspruch 1, dadurch gekennzeichnet, daß man das rohe Endprodukt in Lösung beläßt und zur Reinigung in der gleichen Lösung mit konzentrierter Schwefelsäure als Mineralsäure versetzt.

**Claims**

1. Process for the preparation of phenoxy-azolyl-butanone derivatives of the general formula

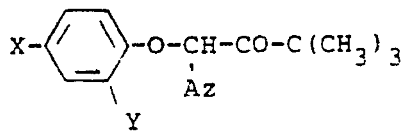

(I)

in which

X represents halogen,

Y represents hydrogen or halogen and

Az represents an imidazolyl radical or 1,2,4-triazolyl radical,

by reacting dichloropinacolin of the formula

$$Cl_2CH—CO—C(CH_3)_3 \qquad (II)$$

with an azole of the general formula

$$Az—H \qquad (III)$$

in which

Az has the meaning given above,

and a phenol of the formula

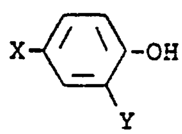

in which

X and Y have the meaning given above,

in the presence of an acid-binding agent, characterised in that the reaction is carried out in the presence of a water-immiscible organic solvent at temperatures between 40 and 150 °C, the crude end product of the formula I is left in solution without changing the solvent, and, for purification of the product, in the same solution, mineral acids are added at temperatures between 0 and + 80 °C, the salt is separated off and the pure end product of the formula I is isolated from the salt in the customary manner.

2. Process according to Claim 1, characterised in that the reaction is carried out in the presence of a water-immiscible ketone.

3. Process according to Claims 1 and 2, characterised in that the reaction is carried out in the presence of methyl isobutyl ketone.

4. Process according to Claim 1, characterised in that the crude end product is left in solution and, for purification of the product, in the same solution, concentrated sulphuric acid is added as the mineral acid.

**Revendications**

1. Procédé de fabrication de dérivés de phénoxy-azolyl-butanone de formule générale

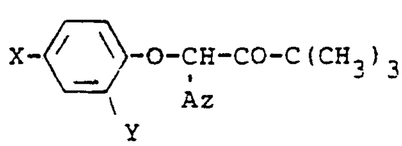

(I)

dans laquelle

X représente de l'halogène,

Y de l'hydrogène ou de l'halogène et

Az un radical imidazolyle ou 1,2,4-triazolyle,

par réaction de la dichloropinacoline de formule

$$Cl_2CH—CO—C(CH_3)_3 \qquad (II)$$

avec un azol de formule générale

Az—H (III)

dans laquelle

Az possède la signification indiquée plus haut

et d'un phénol de formule

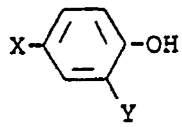

dans laquelle

X et Y possèdent la signification indiquée plus haut,

en présence d'un agent fixateur d'acide, caractérisé en ce qu'on exécute la réaction en présence d'un solvant organique non miscible avec l'eau à des températures entre 40 et 150 °C, on laisse le produit final brut de formule I en solution sans changer de solvant et pour la purification on ajoute dans la même solution à des températures entre 0 et + 180 °C des acides minéraux, et l'on isole à partir du sel séparé le produit final pur de formule I de la manière usuelle.

2. Procédé selon la revendication 1, caractérisé en ce qu'on effectue la réaction en présence d'une cétone non miscible avec l'eau.

3. Procédé selon les revendications 1 et 2, caractérisé en ce qu'on effectue la réaction en présence de méthyl-isobutyl-cétone.

4. Procédé selon la revendication 1, caractérisé en ce qu'on laisse le produit final brut en solution et pour la purification on ajoute dans la même solution de l'acide sulfurique concentré en tant qu'acide minéral.